# EUROPÄISCHE PATENTSCHRIFT

(11) **EP 4 222 491 B1**
(45) Veröffentlichungstag und Bekanntmachung des Hinweises auf die Patenterteilung: **01.01.2025**
(21) Anmeldenummer: 21787319.9
(22) Anmeldetag: 29.09.2021
(51) Int. Cl.: G01N 33/00, A61L 2/20, A61L 2/24, A61L 2/26, B65B 55/10

(54) **VERFAHREN ZU EINER BESTIMMUNG EINER STOFFKONZENTRATION SOWIE ÜBERWACHUNGSVORRICHTUNG ZU EINER ÜBERWACHUNG EINER STOFFKONZENTRATION MITTELS EINES ENTSPRECHENDEN VERFAHRENS**
METHOD FOR DETERMINING A SUBSTANCE CONCENTRATION AND MONITORING APPARATUS FOR MONITORING A SUBSTANCE CONCENTRATION BY MEANS OF A CORRESPONDING METHOD
PROCÉDÉ DE DÉTERMINATION D'UNE CONCENTRATION DE SUBSTANCE ET APPAREIL DE SURVEILLANCE PERMETTANT LA SURVEILLANCE D'UNE CONCENTRATION DE SUBSTANCE AU MOYEN D'UN PROCÉDÉ CORRESPONDANT

(30) Priorität: 29.09.2020 DE 102020125456
(43) Veröffentlichungstag der Anmeldung: 09.08.2023
(73) Patentinhaber: Ampack GmbH, 86343 Königsbrunn (DE)
(72) Erfinder: WEBER, Judith, 52146 Würselen (DE); HAAK, Jürgen, 86343 Königsbrunn (DE); TOMSCHI, Korbinian, 86343 Königsbrunn (DE); KEMPF, Leonie, 86343 Königsbrunn (DE); HEICHELE, Bernhard, 86343 Königsbrunn (DE)
(74) Vertreter: Daub, Thomas
(86) Internationale Anmeldenummer: PCT/EP2021/076777
(87) Internationale Veröffentlichungsnummer: WO 2022/069533

(56) Entgegenhaltungen:
- KUNWAR P SINGH ET AL: "Artificial intelligence based modeling for predicting the disinfection by-products in water", CHEMOMETRICS AND INTELLIGENT LABORATORY SYSTEMS, ELSEVIER SCIENCE PUBLISHERS B.V. AMSTERDAM, NL, vol. 114, 24 March 2012 (2012-03-24), pages 122 - 131, XP028480211, ISSN: 0169-7439, [retrieved on 20120330], DOI: 10.1016/J.CHEMOLAB.2012.03.014
- STEFFEN REISERT ET AL: "Towards a multi-sensor system for the evaluation of aseptic processes employing hydrogen peroxide vapour (H2O2)", PHYSICA STATUS SOLIDI (A), vol. 208, no. 6, 10 June 2011 (2011-06-10), pages 1351 - 1356, XP055067319, ISSN: 1862-6300, DOI: 10.1002/pssa.201001138

## Beschreibung

### Stand der Technik

Es sind bereits Verfahren zu einer Ermittlung eines Verbrauchs von einem Stoff in einem flüssigen Aggregatszustand, insbesondere von H₂O₂ in einem flüssigen Aggregatszustand, bekannt, die bei einem Sterilisationsprozess einer Produktionsmaschine verwendet werden.

Aus DE 603 07 890 T2 ist ein Verfahren mit einem physischen Sensor zur Erfassung einer H₂O₂-Konzentration in einem Gasgemisch in einem Sterilisationsprozess einer Produktionsmaschine bekannt, wobei bei dem aus der DE 603 07 890 T2 bekannten Verfahren die H₂O₂-Konzentration in dem Gasgemisch mittels des physischen Sensors ermittelt wird, um eine zu einer Erzeugung des Gasgemisches in dem Sterilisationsprozess notwendige Menge von zuzugebendem H₂O₂ zu regeln.

Aus DE 602 04 543 T2 und DE 10 2016 125 027 A1 sind ebenfalls jeweils Verfahren mit einem physischen Sensor zur Erfassung einer H₂O₂-Konzentration in einem Gasgemisch in einem Sterilisationsprozess einer Produktionsmaschine bekannt.

Zudem ist bereits ein physischer Sensor, der von der Firma IMAGINE Engineering GmbH unter dem Namen "PeroxGasMonitor" vertrieben wird und der eine Stoffkonzentration, insbesondere eine H₂O₂-Konzentration, in einem Gasgemisch in einem Sterilisationsprozess einer Produktionsmaschine erfassen kann, bekannt. Der physische Sensor ist in einem Rohrleitungssystem einer Produktionsmaschine anordenbar, in dem H₂O₂ in einem gasförmigen Aggregatszustand geführt wird. Der physische Sensor weist ein Messprinzip auf, bei dem H₂O₂ an einem Messkopf des physischen Sensors thermokatalytisch zersetzt wird. Das am Messkopf thermokatalytisch zersetzte H₂O₂ steht für den Sterilisationsprozess nicht mehr zur Verfügung. Ferner weist der physische Sensor eine Öffnung nach außen auf, sodass H₂O₂-Dampf entweichen kann.

STEFFEN REISERT ET AL: "Towards a multi-sensor system for the evaluation of aseptic processes employing hydrogen peroxide vapour (H2O2)",PHYSICA STATUS SOLIDI (A), Bd. 208, Nr. 6, 10. Juni 2011 (2011-06-10), Seiten 1351-1356, XP055067319,ISSN: 1862-6300, DO!: 10.1002/pssa.201001138 schlägt vor neben Gassensordaten auch Prozessparameter in einem Modell maschinellen Lernens zu verarbeiten.

### Offenbarung der Erfindung

Die Erfindung schlägt ein Verfahren, insbesondere ein zumindest im Wesentlichen frei von einem physischen Sensor zur Erfassung einer Stoffkonzentration arbeitendes Verfahren, zu einer Bestimmung einer Stoffkonzentration, insbesondere einer H₂O₂-Konzentration, in einem Gasgemisch in einem Sterilisationsprozess einer Produktionsmaschine vor, wobei in zumindest einem Verfahrensschritt zumindest ein verschieden von der Stoffkonzentration ausgebildeter umgebungs- und/oder prozessspezifischer Parameter mittels zumindest eines Sensors einer Überwachungsvorrichtung der Produktionsmaschine erfasst wird, und wobei in zumindest einem Verfahrensschritt der zumindest eine erfasste umgebungs- und/oder prozessspezifische Parameter einem mittels eines maschinellen Lernprozesses erstellten Auswertemodell zu einer Auswertung zugeführt wird und in Abhängigkeit von der Auswertung auf die Stoffkonzentration, insbesondere auf die H₂O₂-Konzentration, in dem Gasgemisch geschlossen wird. Vorzugsweise ist der mittels eines Sensors der Überwachungsvorrichtung erfasste umgebungs- und/oder prozessspezifische Parameter verschieden von der Stoffkonzentration, insbesondere der H₂O₂-Konzentration, in einem Gasgemisch in dem Sterilisationsprozess ausgebildet. Bevorzugt wird die zu bestimmende Stoffkonzentration, insbesondere die zu bestimmende H₂O₂-Konzentration, im Gasgemisch in dem Sterilisationsprozess mittels des erfindungsgemäßen Verfahrens, indirekt ermittelt, insbesondere nicht direkt mittels eines Sensors erfasst, sondern über einen Zusammenhang der zu bestimmenden Stoffkonzentration, insbesondere der zu bestimmenden H₂O₂-Konzentration, im Gasgemisch mit weiteren umgebungs- und/oder prozessspezifischen Parametern der Produktionsmaschine ermittelt.

Vorzugsweise erfolgt in dem erfindungsgemäßen Verfahren eine Ermittlung der oder ein Rückschluss auf die zu bestimmende/n Stoffkonzentration, insbesondere H₂O₂-Konzentration, im Gasgemisch anhand eines Abgleichs und/oder einer Auswertung des zumindest einen während eines aktuellen Betriebs erfassten umgebungs- und/oder prozessspezifischen Parameters, insbesondere der während eines aktuellen Betriebs erfassten umgebungs- und/oder prozessspezifischen Parameter, mit in dem mittels des maschinellen Lernprozesses erstellten Auswertemodell hinterlegten Daten. Vorzugsweise wird der Zusammenhang der zu bestimmenden Stoffkonzentration, insbesondere der zu bestimmenden H₂O₂-Konzentration, im Gasgemisch mit zumindest einem umgebungs- und/oder prozessspezifischen Parameter, insbesondere mit vielen umgebungs- und/oder prozessspezifischen Parametern, anhand von einer Vielzahl an im Vorfeld erfassten Daten in dem mittels des maschinellen Lernprozesses erstellten Auswertemodell erfasst, insbesondere in einem Datenaufzeichnungsbetrieb. Bevorzugt wird das mittels des maschinellen Lernprozesses erstellte Auswertemodell während eines Betriebs der Produktionsmaschine stetig mit weiteren Daten ergänzt. Denkbar ist, dass in regelmäßigen oder unregelmäßigen zeitlichen Abständen eine Überprüfung von im Auswertemodell abgelegten Datensätzen infolge einer Erfassung von Werten für die Stoffkonzentration, insbesondere der H₂O₂-Konzentration, im Gasgemisch mittels eines physischen Sensors erfolgt, insbesondere um eine hohe Zuverlässigkeit des Verfahrens zu ermöglichen. Das Verfahren bildet vorzugsweise eine Art Softsensor zu einer Bestimmung einer Stoffkonzentration, insbesondere einer H₂O₂-Konzentration, in dem Gasgemisch in dem Sterilisationsprozess der Produktionsmaschine

Das mittels des maschinellen Lernprozesses erstellte Auswertemodell wird vorzugsweise dadurch erstellt, dass während des Datenaufzeichnungsbetriebs unterschiedliche Einstellungen der umgebungs- und/oder prozessspezifischen Parameter erfolgen und deren Auswirkung auf die Stoffkonzentration, insbesondere der H₂O₂-Konzentration, im Gasgemisch mittels eines physischen Sensors zur Erfassung der Stoffkonzentration, insbesondere der H₂O₂-Konzentration, im Gasgemisch aufgezeichnet werden, insbesondere um im realen Betrieb, insbesondere Regelbetrieb, des erfindungsgemäßen Verfahrens, insbesondere in einem frei von einem physischen Sensor zur Erfassung der Stoffkonzentration, insbesondere der H₂O₂-Konzentration, im Gasgemisch ausgebildeten Betrieb des erfindungsgemäßen Verfahrens, zuverlässige Rückschlüsse auf Werte für die Stoffkonzentration, insbesondere der H₂O₂-Konzentration, im Gasgemisch zu erhalten. Vorzugsweise erfolgt der Datenaufzeichnungsbetrieb vor einem realen Betrieb, insbesondere vor einem Regelbetrieb, der Produktionsmaschine, wie beispielsweise während einer Installation der Produktionsmaschine an einem für den realen Betrieb, insbesondere Regelbetrieb, vorgesehenen Standort der Produktionsmaschine. Insbesondere ist der Datenaufzeichnungsbetrieb nur einmal durchzuführen, insbesondere um das Auswertemodell mit Daten zu füllen. Es ist denkbar, dass der Datenaufzeichnungsbetrieb für jede Produktionsmaschine individuell durchgeführt wird oder dass der Datenaufzeichnungsbetrieb lediglich einmal unter vorgegebenen Bedingungen durchgeführt wird und auf verschiedensten Produktionsmaschinen implementierbar ist, wobei beispielsweise infolge einer Netzwerkkommunikation das Auswertemodell weiter angelernt werden kann. Bevorzugt werden zur Erstellung des Auswertemodells die, insbesondere während des Datenaufzeichnungsbetriebs, durchgeführten Einstellungen des/der umgebungs- und/oder prozessspezifische/n Parameter und Werte der Stoffkonzentration, die mittels des physischen Sensors zur Erfassung der Stoffkonzentration, insbesondere der H₂O₂-Konzentration, im Gasgemisch aufgezeichnet werden, in Datensätzen für das Auswertemodell abgelegt. Bevorzugt greift das mittels des maschinellen Lernprozesses erstellte Auswertemodell in einem realen Betrieb, insbesondere Regelbetrieb, der Produktionsmaschine, insbesondere in einem frei von einem physischen Sensor zur Erfassung der Stoffkonzentration ablaufenden Betrieb, auf diese Datensätze zu und/oder gleicht die während eines realen Betriebs, insbesondere Regelbetriebs, der Produktionsmaschine erfassten umgebungs- und/oder prozessspezifischen Parameter mit den in den Datensätzen hinterlegten Werten ab, insbesondere um auf die Stoffkonzentration, insbesondere die H₂O₂-Konzentration, im Gasgemisch in Abhängigkeit von erfassten umgebungs- und/oder prozessspezifischen Parametern zu schließen. Das mittels des maschinellen Lernprozesses erstellte Auswertemodell kann in einer, insbesondere direkt, dem Sterilisationsprozess oder der Produktionsmaschine zugeordneten Recheneinheit, insbesondere einer Recheneinheit der Überwachungsvorrichtung der Produktionsmaschine, wie beispielsweise einer speicherprogrammierbaren Steuerung (SPS) o. dgl. integriert sein, insbesondere als Teil eines Betriebsprogramms, der SPS oder Teil eines netzwerkbasierten Betriebssystems, wie beispielsweise Teil eines cloudbasierten Betriebssystems, ein in einem Edge Device hinterlegten Betriebsprogramm o. dgl.

Vorzugsweise werden für die Ermittlung der oder für den Rückschluss auf die zu bestimmende/n Stoffkonzentration, insbesondere H₂O₂-Konzentration, im Gasgemisch umgebungs- und/oder prozessspezifische Parameter der Produktionsmaschine erfasst und dem mittels des maschinellen Lernprozesses erstellten Auswertemodell zugeführt, die bereits, insbesondere für den Betrieb der Produktionsmaschine sowieso vorhanden oder zu überwachen sind. Der zumindest eine zu erfassende umgebungs- und/oder prozessspezifische Parameter der Produktionsmaschine ist vorzugsweise ein den Sterilisationsprozess oder einen Produktionsprozess der Produktionsmaschine beeinflussender oder definierender Parameter, wie beispielsweise ein Parameter einer die Produktionsmaschine umgebenden Umgebung, insbesondere eine Temperatur, ein Druck o. dgl., ein Parameter eines für den Sterilisationsprozess verwendeten Stoffs, insbesondere in einem flüssigen Aggregatszustand, eine Taktzahl, eine Taktzeit, eine Durchlaufzeit oder ein anderer, einem Fachmann als sinnvoll erscheinender umgebungs- und/oder prozessspezifischer Parameter der Produktionsmaschine. Das erfindungsgemäße Verfahren ist vorzugsweise für einen Einsatz in einem Entkeimungsprozess oder in einem Sterilisationsprozess von Packmitteln in Produktionsmaschinen im Bereich Aseptik und/oder Ultra-Clean vorgesehen. Die Produktionsmaschine kann für die Lebensmittelindustrie oder für die Medizinindustrie vorgesehen sein. Unter "vorgesehen" soll insbesondere speziell eingerichtet, speziell ausgelegt, speziell programmiert und/oder speziell ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Eine Durchführung des Verfahrens kann vorteilhaft frei von zusätzlichen Sensoren zur Erfassung einer Stoffkonzentration erfolgen. Insbesondere erfolgt eine Durchführung des Verfahrens anhand von bereits bei bekannten Produktionsmaschinen eingesetzten Sensoren. Es ist vorteilhaft ohne oder mit geringem Zusatzaufwand eine zuverlässige Bestimmung einer Stoffkonzentration, insbesondere H₂O₂-Konzentration, im Gasgemisch eines Sterilisationsprozesses möglich. Mittels der erfindungsgemäßen Ausgestaltung kann vorteilhaft eine einfache Implementierung auf bereits vorhandenen Produktionsmaschinen erreicht werden, um eine zuverlässige Bestimmung der Stoffkonzentration, insbesondere der H₂O₂-Konzentration, in dem Gasgemisch in dem Sterilisationsprozess zu ermöglichen. Es kann vorteilhaft eine hohe Prozesssicherheit ermöglicht werden, insbesondere da eine Abweichung einer Stoffkonzentration, insbesondere der H₂O₂-Konzentration, in dem Gasgemisch in dem Sterilisationsprozess frühzeitig erkennbar ist. Es kann vorteilhaft eine akustische, optische und/oder haptische Signalausgabe in Abhängigkeit von der bestimmten Stoffkonzentration, insbesondere der H₂O₂-Konzentration, in dem Gasgemisch erfolgen, insbesondere um einen Bediener auf eine zu geringe oder auf eine zu hohe Stoffkonzentration, insbesondere der H₂O₂-Konzentration, in dem Gasgemisch im Sterilisationsprozess hinzuweisen. Es kann vorteilhaft, insbesondere kostengünstig und zeitsparend, eine weitestgehend in Echtzeit erfolgende Bestimmung der Stoffkonzentration, insbesondere der H₂O₂-Konzentration, in dem Gasgemisch im Sterilisationsprozess erfolgen. Es können vorteilhaft mehrere Verdampfervorrichtungen der Produktionsmaschine kostengünstig hinsichtlich der Stoffkonzentration überwacht werden, insbesondere ohne für jede einzelne Verdampfervorrichtung einen einzelnen physischen Sensor zur Erfassung einer Stoffkonzentration an der jeweiligen Verdampfervorrichtung vorsehen zu müssen.

Des Weiteren wird vorgeschlagen, dass in zumindest einem Verfahrensschritt zumindest zwei, insbesondere zumindest vier, verschiedene umgebungs- und/oder prozessspezifische Parameter mittels Sensoren der Überwachungsvorrichtung erfasst werden und dem mittels des maschinellen Lernprozesses erstellten Auswertemodell zur Auswertung zugeführt werden, um in Abhängigkeit von der Auswertung auf die Stoffkonzentration in dem Gasgemisch zu schließen. Es ist jedoch auch denkbar, dass mehr als vier verschiedene umgebungs- und/oder prozessspezifische Parameter mittels Sensoren der Überwachungsvorrichtung erfasst werden und dem mittels des maschinellen Lernprozesses erstellten Auswertemodell zur Auswertung zugeführt werden, um in Abhängigkeit von der Auswertung auf die Stoffkonzentration in dem Gasgemisch zu schließen. Für eine zuverlässige Bestimmung der Stoffkonzentration, insbesondere der H₂O₂-Konzentration, in einem Gasgemisch in dem Sterilisationsprozess werden vorzugsweise mindestens vier verschiedene umgebungs- und/oder prozessspezifische Parameter mittels Sensoren der Überwachungsvorrichtung erfasst und dem mittels des maschinellen Lernprozesses erstellten Auswertemodell zur Auswertung zugeführt. Bevorzugt sind die zumindest zwei, insbesondere zumindest vier, verschiedenen umgebungs- und/oder prozessspezifischen Parameter aus einer Gruppe von Parametern ausgewählt, wobei die Gruppe von Parametern Parameter umfasst, die eine die Produktionsmaschine umgebende Umgebungsluft definieren, die einen flüssigen Stoff, der zur Generierung des Gasgemischs verdampft wird, definieren, die eine zu einer Förderung des flüssigen und/oder aerosolierten Stoffs vorgesehene Druckluft definieren, die eine Verdampfervorrichtung der Produktionsmaschine definieren und/oder die eine an einer Verdampferausgangsleitung der Produktionsmaschine angeordnete Heizeinheit der Produktionsmaschine definieren. Mittels der erfindungsgemäßen Ausgestaltung kann vorteilhaft eine zuverlässige Bestimmung der Stoffkonzentration, insbesondere der H₂O₂-Konzentration, in dem Gasgemisch in dem Sterilisationsprozess frei von einem physischen Sensor zu einer Erfassung der Stoffkonzentration ermöglicht werden. Es kann vorteilhaft eine einfache Implementierung auf bereits vorhandenen Produktionsmaschinen erreicht werden, um eine zuverlässige Bestimmung der Stoffkonzentration, insbesondere der H₂O₂-Konzentration, in dem Gasgemisch in dem Sterilisationsprozess zu ermöglichen.

Ferner wird vorgeschlagen, dass der umgebungs- und/oder prozessspezifische Parameter eine die Produktionsmaschine umgebende Umgebungsluft definierende Kenngröße, insbesondere eine relative Luftfeuchte der Umgebungsluft, eine Temperatur der Umgebungsluft und/oder ein, insbesondere absoluter, Luftdruck der Umgebungsluft, ist, die mittels eines Sensors, insbesondere eines Umgebungsluftfeuchtesensors, eines Umgebungstemperatursensors und/oder eines Umgebungsluftdrucksensors, der Überwachungsvorrichtung erfasst wird. Vorzugsweise wird die relative Luftfeuchte der Umgebungsluft in [%] gemessen. Insbesondere umfasst die Überwachungsvorrichtung zu einer Erfassung der relativen Luftfeuchte der Umgebungsluft zumindest einen als Umgebungsluftfeuchtesensor, insbesondere als Hygrometer, ausgebildeten Sensor. Bevorzugt wird der Luftdruck der Umgebungsluft in [Pa] oder [hPa] gemessen. Vorzugsweise umfasst die Überwachungsvorrichtung zu einer Erfassung des Luftdrucks der Umgebungsluft zumindest einen als Umgebungsluftdrucksensor, insbesondere als Barometer, ausgebildeten Sensor. Bevorzugt wird die Temperatur der Umgebungsluft in [°C] gemessen. Vorzugsweise umfasst die Überwachungsvorrichtung zu einer Erfassung der Temperatur der Umgebungsluft zumindest einen als Umgebungstemperatursensor, insbesondere als Thermometer, ausgebildeten Sensor. Insbesondere ist für eine zuverlässige Bestimmung der Stoffkonzentration, insbesondere der H₂O₂-Konzentration, eine Priorität einer Erfassung der Temperatur der Umgebungsluft und der relativen Luftfeuchte der Umgebungsluft höher als eine Erfassung des Luftdrucks der Umgebungsluft. Mittels der erfindungsgemäßen Ausgestaltung können vorteilhaft umgebungs- und/oder prozessspezifische Parameter zu einer Bestimmung der Stoffkonzentration, insbesondere der H₂O₂-Konzentration, in dem Gasgemisch in dem Sterilisationsprozess herangezogen werden, die einen, insbesondere großen, Einfluss auf die Stoffkonzentration, insbesondere auf einen die Stoffkonzentration hervorrufenden Verdampfervorgang des Stoffs im flüssigen Zustand, haben. Es kann vorteilhaft eine zuverlässige Bestimmung der Stoffkonzentration, insbesondere der H₂O₂-Konzentration, in dem Gasgemisch in dem Sterilisationsprozess frei von einem physischen Sensor zu einer Erfassung der Stoffkonzentration ermöglicht werden. Es kann vorteilhaft eine einfache Implementierung auf bereits vorhandenen Produktionsmaschinen erreicht werden, um eine zuverlässige Bestimmung der Stoffkonzentration, insbesondere der H₂O₂-Konzentration, in dem Gasgemisch in dem Sterilisationsprozess ermöglicht werden. Es können vorteilhaft bereits in einem Realbetrieb der Produktionsmaschine erfasste und überwachte Parameter zu einer Bestimmung der Stoffkonzentration, insbesondere der H₂O₂-Konzentration, in dem Gasgemisch in dem Sterilisationsprozess ermöglicht werden.

Des Weiteren wird vorgeschlagen, dass der umgebungs- und/oder prozessspezifische Parameter eine zu einer Förderung eines flüssigen und/oder aerosolierten Stoffs, der zu einer Generierung des Gasgemischs verdampft wird, vorgesehene Druckluft definierende Kenngröße, insbesondere ein Volumenstrom der Druckluft, eine Temperatur der Druckluft, ein Druck der Druckluft, ein Sättigungsdampfdruck der Druckluft, ein Dampfanteil der Druckluft und/oder eine absolute Luftfeuchte der Druckluft, ist, die mittels eines Sensors, insbesondere eines Druckluftvolumenstromsensors, eines Drucklufttemperatursensors, eines Druckluftdrucksensors, eines Druckluftsättigungsdampfdrucksensors, eines Druckluftdampfanteilsensors und/oder eines Druckluftfeuchtesensors, der Überwachungsvorrichtung erfasst wird. Bevorzugt wird der Volumenstrom der Druckluft in [m³/h] gemessen. Insbesondere umfasst die Überwachungsvorrichtung zu einer Erfassung des Volumenstroms der Druckluft zumindest einen als Druckluftvolumenstromsensor, insbesondere als Volumenstromsensor oder Durchflussmesser, ausgebildeten Sensor. Bevorzugt wird die Temperatur der Druckluft in [°C] gemessen. Vorzugsweise umfasst die Überwachungsvorrichtung zu einer Erfassung der Temperatur der Druckluft zumindest einen als Drucklufttemperatursensor, insbesondere als Thermometer, ausgebildeten Sensor. Bevorzugt wird der Luftdruck der Druckluft in [Pa] oder [hPa] gemessen. Vorzugsweise umfasst die Überwachungsvorrichtung zu einer Erfassung des Luftdrucks der Druckluft zumindest einen als Druckluftdrucksensor, insbesondere als Manometer, ausgebildeten Sensor. Bevorzugt wird der Sättigungsdampfdruck der Druckluft in [Pa] oder [hPa] gemessen. Vorzugsweise umfasst die Überwachungsvorrichtung zu einer Erfassung des Sättigungsdampfdrucks der Druckluft zumindest einen als Druckluftsättigungsdampfdrucksensor, insbesondere als Taupunktsensor, ausgebildeten Sensor. Bevorzugt wird der Dampfanteil der Druckluft in [ppmV/V] gemessen. Vorzugsweise umfasst die Überwachungsvorrichtung zu einer Erfassung des Dampfanteils der Druckluft zumindest einen als Druckluftdampfanteilsensor, insbesondere als Taupunktsensor, ausgebildeten Sensor. Insbesondere wird die absolute Luftfeuchte der Druckluft in [g/m³] gemessen. Vorzugsweise umfasst die Überwachungsvorrichtung zu einer Erfassung der absoluten Luftfeuchte der Druckluft zumindest einen als Druckluftfeuchtesensor, insbesondere als Taupunktsensor, ausgebildeten Sensor. Insbesondere ist für eine zuverlässige Bestimmung der Stoffkonzentration, insbesondere der H₂O₂-Konzentration, eine Priorität einer Erfassung des Drucks der Druckluft, des Sättigungsdampfdrucks der Druckluft und des Dampfanteils der Druckluft höher als eine Erfassung des Volumenstroms der Druckluft, der Temperatur der Druckluft sowie der absoluten Luftfeuchte der Druckluft. Mittels der erfindungsgemäßen Ausgestaltung können vorteilhaft bereits in einem Realbetrieb der Produktionsmaschine erfasste und überwachte Parameter zu einer Bestimmung der Stoffkonzentration, insbesondere der H₂O₂-Konzentration, in dem Gasgemisch in dem Sterilisationsprozess ermöglicht werden. Es können vorteilhaft umgebungs- und/oder prozessspezifische Parameter zu einer Bestimmung der Stoffkonzentration, insbesondere der H₂O₂-Konzentration, in dem Gasgemisch in dem Sterilisationsprozess herangezogen werden, die einen, insbesondere großen, Einfluss, auf die Stoffkonzentration, insbesondere auf einen die Stoffkonzentration hervorrufenden Verdampfervorgang des Stoffs im flüssigen Zustand, haben. Es kann vorteilhaft eine zuverlässige Bestimmung der Stoffkonzentration, insbesondere der H₂O₂-Konzentration, in dem Gasgemisch in dem Sterilisationsprozess frei von einem physischen Sensor zu einer Erfassung der Stoffkonzentration ermöglicht werden. Es kann vorteilhaft eine einfache Implementierung auf bereits vorhandenen Produktionsmaschinen erreicht werden, um eine zuverlässige Bestimmung der Stoffkonzentration, insbesondere der H₂O₂-Konzentration, in dem Gasgemisch in dem Sterilisationsprozess ermöglicht werden.

Weiterhin wird vorgeschlagen, dass der umgebungs- und/oder prozessspezifische Parameter eine einen flüssigen Stoff, der zur Generierung des Gasgemischs verdampft wird, definierende Kenngröße, insbesondere eine Konzentration des Stoffs im flüssigen Zustand, eine Temperatur des Stoffs im flüssigen Zustand und/oder eine Menge/Volumen des Stoffs im flüssigen Zustand pro Takt des Sterilisationsprozesses, ist, die mittels eines Sensors, insbesondere eines Stoffkonzentrationssensors, eines Stofftemperatursensors und/oder eines Stoffmengensensors, der Überwachungsvorrichtung erfasst wird. Bevorzugt wird die Konzentration des Stoffs im flüssigen Zustand in [%] oder in [mol/m³] gemessen. Vorzugsweise wird Konzentration des Stoffs im flüssigen Zustand in % aus der Dichte in [g/cm³] und der Temperatur in [°C] des Stoffs im flüssigen Zustand bestimmt, insbesondere über ein Nomogramm. Insbesondere umfasst die Überwachungsvorrichtung zu einer Erfassung der Konzentration des Stoffs im flüssigen Zustand zumindest einen als Stoffkonzentrationssensor ausgebildeten Sensor. Bevorzugt wird die Temperatur des Stoffs im flüssigen Zustand in [°C] gemessen. Vorzugsweise umfasst die Überwachungsvorrichtung zu einer Erfassung der Temperatur des Stoffs im flüssigen Zustand zumindest einen als Stofftemperatursensor, insbesondere als Thermometer, ausgebildeten Sensor. Bevorzugt wird die Menge/das Volumen des Stoffs im flüssigen Zustand, insbesondere pro Takt des Sterilisationsprozesses, in [ml] gemessen. Vorzugsweise umfasst die Überwachungsvorrichtung zu einer Erfassung der Menge/des Volumens des Stoffs im flüssigen Zustand, insbesondere pro Takt des Sterilisationsprozesses, zumindest einen als Stoffmengensensor, insbesondere als Durchflusssensor, ausgebildeten Sensor. Es ist jedoch auch denkbar, dass die Menge/das Volumen des Stoffs im flüssigen Zustand, insbesondere pro Takt des Sterilisationsprozesses, infolge einer Nutzung eins Gefäßes, insbesondere eines Schöpfgefäßes, das bei jedem Takt des Sterilisationsprozesses eine vorgegebene Menge/ein vorgegebenes Volumen des Stoffs im flüssigen Zustand aufnimmt, zu einer Erfassung der Menge/des Volumens des Stoffs im flüssigen Zustand, insbesondere pro Takt des Sterilisationsprozesses, erfasst wird. Insbesondere ist für eine zuverlässige Bestimmung der Stoffkonzentration, insbesondere der H₂O₂-Konzentration, eine Priorität einer Erfassung der Menge/des Volumens des Stoffs im flüssigen Zustand und die Temperatur des Stoffs im flüssigen Zustand höher als eine Erfassung der Konzentration des Stoffs im flüssigen Zustand. Mittels der erfindungsgemäßen Ausgestaltung können vorteilhaft umgebungs- und/oder prozessspezifische Parameter zu einer Bestimmung der Stoffkonzentration, insbesondere der H₂O₂-Konzentration, in dem Gasgemisch in dem Sterilisationsprozess herangezogen werden, die einen, insbesondere großen, Einfluss, auf die Stoffkonzentration, insbesondere auf einen die Stoffkonzentration hervorrufenden Verdampfervorgang des Stoffs im flüssigen Zustand, haben. Es kann vorteilhaft eine zuverlässige Bestimmung der Stoffkonzentration, insbesondere der H₂O₂-Konzentration, in dem Gasgemisch in dem Sterilisationsprozess frei von einem physischen Sensor zu einer Erfassung der Stoffkonzentration ermöglicht werden. Es kann vorteilhaft eine einfache Implementierung auf bereits vorhandenen Produktionsmaschinen erreicht werden, um eine zuverlässige Bestimmung der Stoffkonzentration, insbesondere der H₂O₂-Konzentration, in dem Gasgemisch in dem Sterilisationsprozess zu ermöglichen. Es können vorteilhaft bereits in einem Realbetrieb der Produktionsmaschine erfasste und überwachte Parameter zu einer Bestimmung der Stoffkonzentration, insbesondere der H₂O₂-Konzentration, in dem Gasgemisch in dem Sterilisationsprozess ermöglicht werden.

Zudem wird vorgeschlagen, dass der umgebungs- und/oder prozessspezifische Parameter eine eine Verdampfervorrichtung der Produktionsmaschine definierende Kenngröße, insbesondere eine Temperatur einer Verdampferheizeinheit der Verdampfervorrichtung, insbesondere einer Verdampferspitze der Verdampferheizeinheit, und/oder eine Einschaltdauer der Verdampfervorrichtung, vorzugsweise der Verdampferheizeinheit, ist, die mittels eines Sensors, insbesondere eines Verdampferheizeinheittemperatursensors und/oder eines Verdampfereinschaltdauersensors, der Überwachungsvorrichtung erfasst wird. Die Verdampferheizeinheit kann eine kegelartige Grundform oder eine scheibenartige Grundform aufweisen. Bei einer kegelartigen Grundform der Verdampferheizeinheit weist die Verdampferheizeinheit vorzugsweise eine Heizfläche auf, die als Mantelfläche eines Kegels ausgebildet ist. Bei einer kegelartigen Grundform der Verdampferheizeinheit weist die Verdampferheizeinheit vorzugsweise eine Verdampferspitze auf, deren Temperatur mittels der Überwachungsvorrichtung erfasst wird. Bei einer scheibenartigen Grundform der Verdampferheizeinheit weist die Verdampferheizeinheit eine zumindest im Wesentlichen planare Heizfläche auf, deren Temperatur mittels der Überwachungsvorrichtung erfasst wird. Bevorzugt wird die Temperatur der Verdampferheizeinheit in [°C] gemessen. Vorzugsweise umfasst die Überwachungsvorrichtung zu einer Erfassung der Temperatur der Verdampferheizeinheit zumindest einen als Verdampferheizeinheittemperatursensor, insbesondere als Thermometer, ausgebildeten Sensor. Es ist jedoch auch denkbar, dass die Temperatur der Verdampferheizeinheit alternativ oder zusätzlich zu einer Erfassung mit dem Verdampferheizeinheittemperatursensor aus einer Steuer- oder Regelelektronik, die der Verdampferheizeinheit direkt zugeordnet ist, entnehmbar ist. Bevorzugt wird die Einschaltdauer der Verdampfervorrichtung, insbesondere der Verdampferheizeinheit, in [%] gemessen. Vorzugsweise umfasst die Überwachungsvorrichtung zu einer Erfassung einer Zeit zur Bestimmung der Einschaltdauer der Verdampferheizeinheit zumindest einen als Verdampfereinschaltdauersensor ausgebildeten Sensor. Vorzugsweise weist die Verdampferheizeinheit zumindest einen Heizkreis, wie beispielsweise eine Heizpatrone, einen Heizwiderstand o. dgl., mit integriertem Temperatursensor auf. Es ist jedoch auch denkbar, dass die Verdampferheizeinheit eine Vielzahl an Heizkreisen aufweist, deren Temperaturen individuell erfassbar sind. Die Verdampferheizeinheit, insbesondere der zumindest eine Heizkreis der Verdampferheizeinheit, kennt vorzugsweise zwei Zustände, "an" und "aus". Durch an- und ausschalten der Verdampferheizeinheit wird bevorzugt die Temperatur der Verdampfervorrichtung, insbesondere der Verdampferheizeinheit, geregelt und auf einem gewünschten Einstellwert gehalten. Die Einschaltdauer in [%] gibt an, wie lange eine Heizeinheit, insbesondere die Verdampferheizeinheit, insbesondere der zumindest eine Heizkreis der Verdampferheizeinheit, in der letzten Minute aktiviert war. War die Verdampferheizeinheit, insbesondere der zumindest eine Heizkreis der Verdampferheizeinheit, beispielsweise in der letzten Minute für 20s an, liegt die Einschaltdauer bei 33 % (20/60*100 %). Insbesondere ist für eine zuverlässige Bestimmung der Stoffkonzentration, insbesondere der H₂O₂-Konzentration, eine Priorität einer Erfassung der Einschaltdauer der Verdampfervorrichtung höher als eine Erfassung der Temperatur der Verdampferheizeinheit. Mittels der erfindungsgemäßen Ausgestaltung können vorteilhaft bereits in einem Realbetrieb der Produktionsmaschine erfasste und überwachte Parameter zu einer Bestimmung der Stoffkonzentration, insbesondere der H₂O₂-Konzentration, in dem Gasgemisch in dem Sterilisationsprozess ermöglicht werden. Es kann vorteilhaft eine zuverlässige Bestimmung der Stoffkonzentration, insbesondere der H₂O₂-Konzentration, in dem Gasgemisch in dem Sterilisationsprozess frei von einem physischen Sensor zu einer Erfassung der Stoffkonzentration ermöglicht werden. Es kann vorteilhaft eine einfache Implementierung auf bereits vorhandenen Produktionsmaschinen erreicht werden, um eine zuverlässige Bestimmung der Stoffkonzentration, insbesondere der H₂O₂-Konzentration, in dem Gasgemisch in dem Sterilisationsprozess zu ermöglichen.

Des Weiteren wird vorgeschlagen, dass der umgebungs- und/oder prozessspezifische Parameter eine eine an einer Verdampferausgangsleitung der Produktionsmaschine angeordnete Heizeinheit der Produktionsmaschine definierende Kenngröße, insbesondere eine Temperatur der Heizeinheit und/oder eine Einschaltdauer der Heizeinheit, ist, die mittels eines Sensors, insbesondere eines Heizeinheitentemperatursensors und/oder eines Heizeinheiteneinschaltdauersensors, der Überwachungsvorrichtung erfasst wird. Die Verdampferausgangsleitung ist vorzugsweise dazu vorgesehen, einen Verdampferraum der Verdampfervorrichtung, in dem der flüssige Stoff zur Generierung des Gasgemischs, insbesondere eines H₂O₂-Dampf/Luftgemischs, mittels der Verdampferheizeinheit verdampft wird, mit einer Sterilisationsdüse oder einem Verteiler zu verbinden, insbesondere um das Gasgemisch, insbesondere das H₂O₂-Dampf/Luftgemisch, auf eine, einem Fachmann bereits bekannte Art und Weise zumindest einem Packmittel zu einer Entkeimung zuzuführen. Die an der Verdampferausgangsleitung angeordnete Heizeinheit kann beispielsweise als Wickelheizung, die, insbesondere spiralförmig, um die Verdampferausgangsleitung gewickelt ist, oder als Heizschlauch, der die Verdampferausgangsleitung zumindest in einem Abschnitt der Verdampferausgangsleitung zumindest im Wesentlichen vollständig umgibt, ausgebildet sein. Bevorzugt wird die Temperatur der Heizeinheit in [°C] gemessen. Vorzugsweise umfasst die Überwachungsvorrichtung zu einer Erfassung der Temperatur der Heizeinheit zumindest einen als Heizeinheitentemperatursensor, insbesondere als Thermometer, ausgebildeten Sensor. Es ist jedoch auch denkbar, dass die Temperatur der Heizeinheit alternativ oder zusätzlich zu einer Erfassung mit dem Heizeinheitentemperatursensor aus einer Steuer- oder Regelelektronik, die der Heizeinheit direkt zugeordnet ist, entnehmbar ist. Bevorzugt wird die Einschaltdauer der Heizeinheit in [%] gemessen. Vorzugsweise umfasst die Überwachungsvorrichtung zumindest zu einer Erfassung einer Zeit zur Bestimmung der Einschaltdauer der Heizeinheit zumindest einen als Heizeinheiteneinschaltdauersensor ausgebildeten Sensor. Es ist jedoch auch denkbar, dass das Verfahren unabhängig von einer Erfassung der an der Verdampferausgangsleitung angeordneten Heizeinheit definierenden Kenngröße angewandt wird, insbesondere, da die an der Verdampferausgangsleitung angeordnete Heizeinheit einen geringen Einfluss, insbesondere einen geringeren Einfluss als die zuvor aufgeführten Parameter, auf die Bestimmung der Stoffkonzentration, insbesondere der H₂O₂-Konzentration, in dem Gasgemisch in dem Sterilisationsprozess aufweist. Mittels der erfindungsgemäßen Ausgestaltung können vorteilhaft bereits in einem Realbetrieb der Produktionsmaschine erfasste und überwachte Parameter zu einer Bestimmung der Stoffkonzentration, insbesondere der H₂O₂-Konzentration, in dem Gasgemisch in dem Sterilisationsprozess ermöglicht werden. Es kann vorteilhaft eine zuverlässige Bestimmung der Stoffkonzentration, insbesondere der H₂O₂-Konzentration, in dem Gasgemisch in dem Sterilisationsprozess frei von einem physischen Sensor zu einer Erfassung der Stoffkonzentration ermöglicht werden.

Ferner wird vorgeschlagen, dass in zumindest einem Verfahrensschritt, insbesondere vor einem Regelbetrieb der Produktionsmaschine, eine Kalibration, insbesondere des Auswertemodells, mittels eines, insbesondere physischen, Stoffkonzentrationssensors, insbesondere mittels eines H₂O₂-Konzentrationssensors, erfolgt. Vorzugsweise erfolgt eine Kalibration nach einer Implementierung des Verfahrens, insbesondere des Auswertemodells, in einer Recheneinheit der Produktionsmaschine, insbesondere um das Auswertemodell an die Produktionsmaschine anzupassen und/oder eventuelle Abweichungen zu erkennen. Es ist alternativ oder zusätzlich denkbar, dass die Kalibration nach einem Wartungsbetrieb der Produktionsmaschine durchgeführt wird, insbesondere um eine zuverlässige Bestimmung der Stoffkonzentration, insbesondere der H₂O₂-Konzentration, im Gasgemisch über eine zumindest im Wesentlichen gesamte Lebenszeit der Produktionsmaschine zu ermöglichen. Es ist denkbar, dass in einem alternativen oder zusätzlichen Verfahrensschritt des Verfahrens eine Justierung, insbesondere anhand der Kalibration, des Auswertemodells, insbesondere der hinterlegten Datensätze, erfolgt. Mittels der erfindungsgemäßen Ausgestaltung kann vorteilhaft eine zuverlässige Bestimmung der Stoffkonzentration, insbesondere der H₂O₂-Konzentration, in dem Gasgemisch in dem Sterilisationsprozess ermöglicht werden. Es kann vorteilhaft eine hohe Prozesssicherheit ermöglicht werden.

Zudem geht die Erfindung aus von einer Überwachungsvorrichtung zu einer Überwachung einer Stoffkonzentration, insbesondere einer H₂O₂-Konzentration, in einem Gasgemisch in einem Sterilisationsprozess einer Produktionsmaschine, mit zumindest einer Recheneinheit zu einem Ausführen des Verfahrens nach einem der vorhergehenden Ansprüche und mit zumindest einem Sensor zu einem Erfassen zumindest eines, insbesondere verschieden von der Stoffkonzentration ausgebildeten, umgebungs- und/oder prozessspezifischen Parameters, der zu einem Übertragen von Daten mit der Recheneinheit verbunden ist. Es wird vorgeschlagen, dass die Recheneinheit zumindest einen Speicher aufweist, in dem ein mittels eines maschinellen Lernprozesses erstelltes, insbesondere das bereits zuvor genannte, Auswertemodell abgelegt ist, das dazu eingerichtet ist, in Abhängigkeit von einer Auswertung des zumindest einen erfassten umgebungs- und/oder prozessspezifischen Parameters auf die Stoffkonzentration, insbesondere auf die H₂O₂-Konzentration, in dem Gasgemisch zu schließen, insbesondere frei von einem physischen Stoffkonzentrationssensor. Die Überwachungsvorrichtung umfasst vorzugsweise eine Vielzahl an Sensoren, insbesondere die bereits zuvor genannten Sensoren, zu einer Erfassung der umgebungs- und/oder prozessspezifischen Parameter. Die Sensoren sind vorzugsweise alle drahtgebunden oder drahtlos mit der Recheneinheit datentechnisch verbunden. Vorzugsweise werden die mittels der Sensoren erfassten Daten dem mittels eines maschinellen Lernprozesses erstellten Auswertemodell zu einer Auswertung und/oder Speicherung zugeführt, wobei in Abhängigkeit von der Auswertung der erfassten umgebungs- und/oder prozessspezifischen Parameter auf die Stoffkonzentration, insbesondere auf die H₂O₂-Konzentration, in dem Gasgemisch geschlossen wird. Die Überwachungsvorrichtung kann ein Teil einer Hauptrecheneinheit, insbesondere der SPS, der Produktionsmaschine sein oder separat dazu ausgebildet sein. Mittels der erfindungsgemäßen Ausgestaltung kann vorteilhaft eine hohe Prozesssicherheit ermöglicht werden, insbesondere da eine Abweichung einer Stoffkonzentration, insbesondere der H₂O₂-Konzentration, in dem Gasgemisch in dem Sterilisationsprozess frühzeitig erkennbar ist. Es können vorteilhaft mehrere Verdampfervorrichtungen der Produktionsmaschine kostengünstig hinsichtlich der Stoffkonzentration überwacht werden, insbesondere ohne für jede einzelne Verdampfervorrichtung einen einzelnen physischen Sensor zur Erfassung einer Stoffkonzentration an der jeweiligen Verdampfervorrichtung vorsehen zu müssen.

Des Weiteren wird eine Produktionsmaschine, insbesondere eine Lebensmittelabfüll- und/oder Lebensmittelverpackungsmaschine mit zumindest einer erfindungsgemäßen Überwachungsvorrichtung vorgeschlagen. Mittels der erfindungsgemäßen Ausgestaltung kann vorteilhaft eine hohe Prozesssicherheit, insbesondere in Bezug auf eine Entkeimung von mittels der Produktionsmaschine herstellbaren, verschließbaren und/oder füllbaren Packmitteln, ermöglicht werden. Es können vorteilhaft mehrere Verdampfervorrichtungen der Produktionsmaschine kostengünstig hinsichtlich der Stoffkonzentration überwacht werden, insbesondere ohne für jede einzelne Verdampfervorrichtung einen einzelnen physischen Sensor zur Erfassung einer Stoffkonzentration an der jeweiligen Verdampfervorrichtung vorsehen zu müssen.

Das erfindungsgemäße Verfahren, die erfindungsgemäße Überwachungsvorrichtung und/oder die erfindungsgemäße Produktionsmaschine sollen/soll hierbei nicht auf die oben beschriebene Anwendung und Ausführungsform beschränkt sein. Insbesondere können/kann das erfindungsgemäße Verfahren, die erfindungsgemäße Überwachungsvorrichtung und/oder die erfindungsgemäße Produktionsmaschine zu einer Erfüllung einer hierin beschriebenen Funktionsweise eine von einer hierin genannten Anzahl von einzelnen Elementen, Bauteilen und Einheiten sowie Verfahrensschritten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

### Zeichnung

Weitere Vorteile ergeben sich aus der folgenden Zeichnungsbeschreibung. In der Zeichnung ist ein Ausführungsbeispiel der Erfindung dargestellt. Die Zeichnung, die Beschreibung und die Ansprüche enthalten zahlreiche Merkmale in Kombination.

Es zeigen:
- Fig. 1: eine schematische Darstellung einer erfindungsgemäßen Produktionsmaschine mit einer erfindungsgemäßen Überwachungsvorrichtung zu einer Überwachung einer Stoffkonzentration, insbesondere einer H₂O₂-Konzentration, in einem Gasgemisch in einem Sterilisationsprozess der erfindungsgemäßen Produktionsmaschine,
- Fig. 2: eine schematische Darstellung der erfindungsgemäßen Überwachungsvorrichtung sowie einer Verdampfervorrichtung der erfindungsgemäßen Produktionsmaschine und
- Fig. 3: einen schematischen Ablauf eines erfindungsgemäßen Verfahrens zu einer Bestimmung der Stoffkonzentration, insbesondere der H₂O₂-Konzentration, in dem Gasgemisch in dem Sterilisationsprozess der erfindungsgemäßen Produktionsmaschine.

### Beschreibung des Ausführungsbeispiels

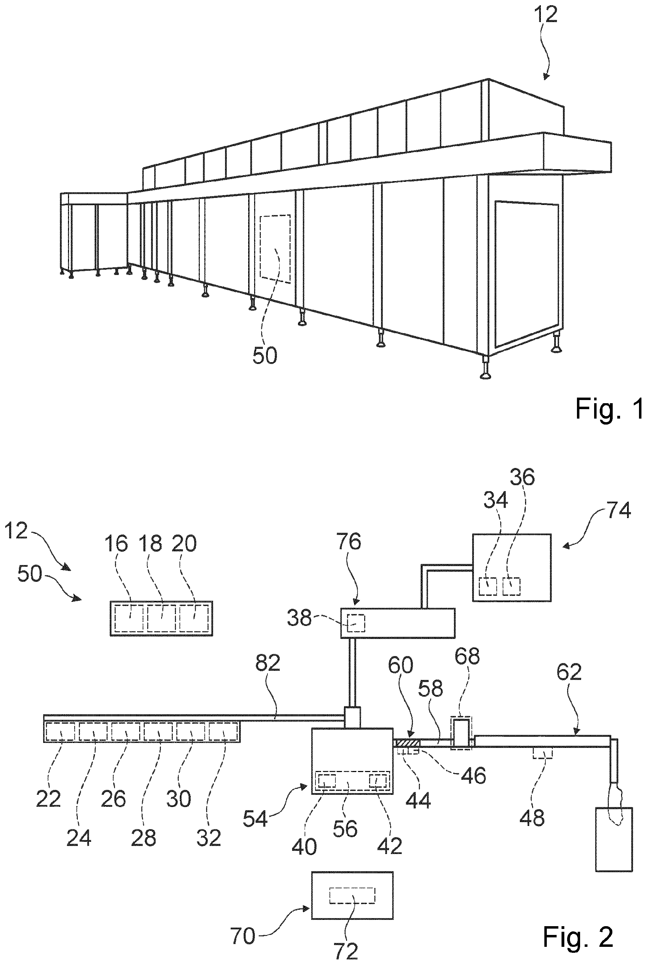

Figur 1 zeigt eine Produktionsmaschine 12, insbesondere eine Lebensmittelabfüll- und/oder Lebensmittelverpackungsmaschine. Es ist jedoch auch denkbar, dass die Produktionsmaschine 12 als Pharmaabfüll- und/oder Pharmaverpackungsanlage ausgebildet ist. Die Produktionsmaschine 12 umfasst vorzugsweise zumindest eine Verdampfervorrichtung 54 zu einem Verdampfen eines Stoffs in flüssigem und/oder in aerosoliertem Zustand, insbesondere von flüssigem H₂O₂ und/oder aerosoliertem H₂O₂. Die Verdampfervorrichtung 54 ist vorzugsweise als Sterilisationsverdampfervorrichtung ausgebildet, die zu einem Einsatz in einem Sterilisationsprozess der Produktionsmaschine 12 vorgesehen ist. Die Verdampfervorrichtung 54 weist zumindest zum Großteil, insbesondere vollständig, eine Funktionsweise und/oder eine Ausgestaltung auf, die einem Fachmann bereits bekannt ist.

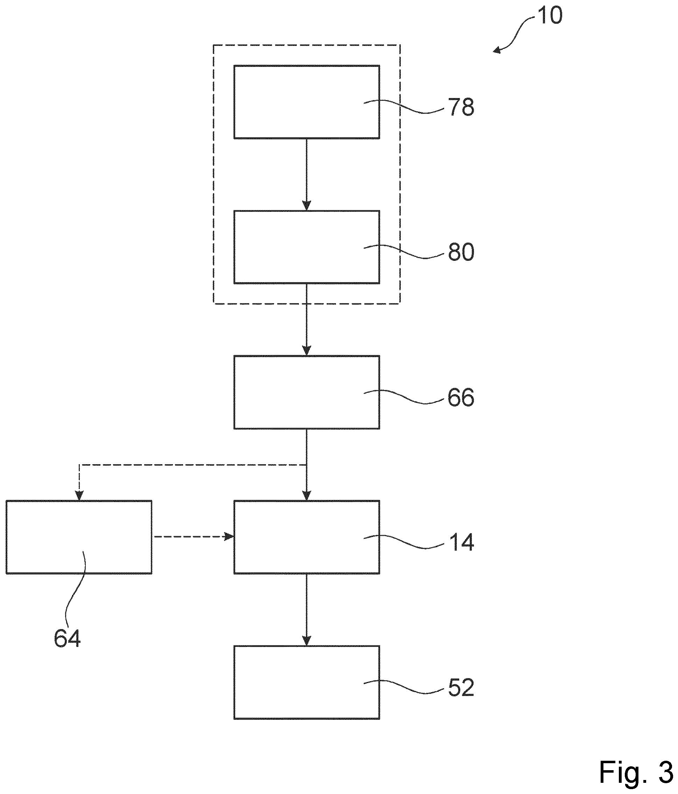

Die Produktionsmaschine 12 weist zumindest eine Überwachungsvorrichtung 50 zu einer Überwachung einer Stoffkonzentration, insbesondere einer H₂O₂-Konzentration, in einem Gasgemisch in dem Sterilisationsprozess der Produktionsmaschine 12 auf. Bevorzugt ist die Überwachungsvorrichtung 50 zu einer Überwachung einer Stoffkonzentration, insbesondere einer H₂O₂-Konzentration, in einem Gasgemisch in dem Sterilisationsprozess in einem Regelbetrieb der Produktionsmaschine 12 frei von einem physischen Stoffkonzentrationssensor 68, insbesondere frei von einem H₂O₂-Konzentrationssensor, ausgebildet. Die Überwachungsvorrichtung 50 umfasst zumindest eine Recheneinheit 70 zu einem Ausführen eines Verfahrens 10 (vgl. Figur 3) zu einer Bestimmung einer Stoffkonzentration, insbesondere einer H₂O₂-Konzentration, in dem Gasgemisch in dem Sterilisationsprozess. Die Recheneinheit 70 weist zumindest einen Speicher 72 auf, in dem ein mittels eines maschinellen Lernprozesses erstelltes Auswertemodell abgelegt ist, das dazu eingerichtet ist, in Abhängigkeit von einer Auswertung zumindest eines erfassten umgebungs- und/oder prozessspezifischen Parameters, insbesondere von einer Vielzahl an erfassten umgebungsund/oder prozessspezifischen Parameter, auf die Stoffkonzentration, insbesondere auf die H₂O₂-Konzentration, in dem Gasgemisch zu schließen, insbesondere frei von dem physischen Stoffkonzentrationssensor 68.

Die Überwachungsvorrichtung 50 umfasst zumindest einen Sensor 16, 18, 20, 22, 24, 26, 28, 30, 32, 34, 36, 38, 40, 42, 44, 46, 48 (vgl. Figur 2) zu einem Erfassen zumindest eines, insbesondere verschieden von der Stoffkonzentration ausgebildeten, umgebungs- und/oder prozessspezifischen Parameters, wobei der Sensor 16, 18, 20, 22, 24, 26, 28, 30, 32, 34, 36, 38, 40, 42, 44, 46, 48 zu einem Übertragen von Daten mit der Recheneinheit 70 verbunden ist. Vorzugsweise umfasst die Überwachungsvorrichtung 50 eine Vielzahl an Sensoren 16, 18, 20, 22, 24, 26, 28, 30, 32, 34, 36, 38, 40, 42, 44, 46, 48 (vgl. Figur 2) zu einem Erfassen mehrerer, insbesondere verschieden von der Stoffkonzentration ausgebildeter, umgebungs- und/oder prozessspezifischer Parameter, wobei die Sensoren 16, 18, 20, 22, 24, 26, 28, 30, 32, 34, 36, 38, 40, 42, 44, 46, 48 zu einem Übertragen von Daten mit der Recheneinheit 70 verbunden sind. Die Sensoren 16, 18, 20, 22, 24, 26, 28, 30, 32, 34, 36, 38, 40, 42, 44, 46, 48 können leitungsgebunden oder drahtlos mit der Recheneinheit 70 zu einer Übertragung von Daten verbunden sein. Die Sensoren 16, 18, 20, 22, 24, 26, 28, 30, 32, 34, 36, 38, 40, 42, 44, 46, 48 sind bevorzugt als physische Sensoren ausgebildet. Die Sensoren 16, 18, 20, 22, 24, 26, 28, 30, 32, 34, 36, 38, 40, 42, 44, 46, 48 der Überwachungsvorrichtung 50 können auf eine, einem Fachmann bereits bekannte Art und Weise alle einzeln ausgebildet sein oder zumindest teilweise kombiniert ausgebildet sein, um die entsprechenden Kenngrößen zu erfassen.

Die Überwachungsvorrichtung 50 umfasst zumindest einen Sensor 16, 18, 20, insbesondere eine Vielzahl an Sensoren 16, 18, 20, zu einer Erfassung einer einer die Produktionsmaschine 12 umgebenden Umgebungsluft definierenden Kenngröße. Insbesondere ist der Sensor 16, 18, 20, insbesondere die Sensoren 16, 18, 20, zu einer Erfassung der Kenngröße, die die Umgebungsluft definieren, an einer Außenseite der Produktionsmaschine 12 und/oder in einem Raum, insbesondere an einer Raumwand des Raums, in dem die Produktionsmaschine 12 installiert ist, angeordnet. Bevorzugt ist zumindest ein Sensor 16 der Überwachungsvorrichtung 50 als Umgebungsluftfeuchtesensor ausgebildet, der zu einem Erfassen einer relativen Luftfeuchte einer die Produktionsmaschine 12 umgebenden Umgebungsluft vorgesehen ist. Vorzugsweise ist zumindest ein Sensor 18 der Überwachungsvorrichtung 50 als Umgebungstemperatursensor ausgebildet, der zu einem Erfassen einer Temperatur der die Produktionsmaschine 12 umgebenden Umgebungsluft vorgesehen ist. Insbesondere ist zumindest ein Sensor 20 der Überwachungsvorrichtung 50 als Umgebungsluftdrucksensor ausgebildet, der zu einem Erfassen eines Luftdrucks der die Produktionsmaschine 12 umgebenden Umgebungsluft vorgesehen ist.

Die Überwachungsvorrichtung 50 umfasst zumindest einen Sensor 22, 24, 26, 28, 30, 32, insbesondere eine Vielzahl an Sensoren 22, 24, 26, 28, 30, 32, zu einer Erfassung einer eine zu einer Förderung eines flüssigen und/oder aerosolierten Stoffs, der zu einer Generierung des Gasgemischs verdampft wird, vorgesehene Druckluft definierenden Kenngröße. Insbesondere ist der Sensor 22, 24, 26, 28, 30, 32, insbesondere die Sensoren 22, 24, 26, 28, 30, 32, zu einer Erfassung der die zu einer Förderung eines flüssigen und/oder aerosolierten Stoffs, der zu einer Generierung des Gasgemischs verdampft wird, vorgesehene Druckluft definierenden Kenngröße an einer Druckluftleitung 82 und/oder an einem Druckerzeuger (hier nicht näher dargestellt) der Produktionsmaschine 12 angeordnet. Bevorzugt ist zumindest ein Sensor 22 der Überwachungsvorrichtung 50 als Druckluftvolumenstromsensor ausgebildet, der zu einem Erfassen eines Volumenstroms der Druckluft vorgesehen ist. Vorzugsweise ist zumindest ein Sensor 24 der Überwachungsvorrichtung 50 als Drucklufttemperatursensor ausgebildet, der zu einem Erfassen einer Temperatur der Druckluft vorgesehen ist. Insbesondere ist zumindest ein Sensor 26 der Überwachungsvorrichtung 50 als Druckluftdrucksensor ausgebildet, der zu einem Erfassen eines Drucks der Druckluft vorgesehen ist. Bevorzugt ist zumindest ein Sensor 28 der Überwachungsvorrichtung 50 als Druckluftsättigungsdampfdrucksensor ausgebildet, der zu einem Erfassen eines Sättigungsdampfdrucks der Druckluft vorgesehen ist. Vorzugsweise ist zumindest ein Sensor 30 der Überwachungsvorrichtung 50 als Druckluftdampfanteilsensor ausgebildet, der zu einem Erfassen eines Dampfanteils der Druckluft vorgesehen ist. Insbesondere ist zumindest ein Sensor 32 der Überwachungsvorrichtung 50 als Druckluftfeuchtesensor ausgebildet, der zu einem Erfassen einer absoluten Luftfeuchte der Druckluft vorgesehen ist.

Die Überwachungsvorrichtung 50 umfasst zumindest einen Sensor 34, 36, 38, insbesondere eine Vielzahl an Sensoren 34, 36, 38, zu einer Erfassung einer einen flüssigen Stoff, der zur Generierung des Gasgemischs verdampft wird, definierenden Kenngröße. Insbesondere ist der Sensor 34, 36, 38, insbesondere die Sensoren 34, 36, 38, zu einer Erfassung der den flüssigen Stoff, der zur Generierung des Gasgemischs verdampft wird, definierenden Kenngröße an einer Vorratseinheit 74 der Produktionsmaschine 12 zu einem Bevorraten des flüssigen Stoffs und/oder an einer Dosiereinheit 76 der Produktionsmaschine 12 zu einem Dosieren des flüssigen Stoffs angeordnet. Die Vorratseinheit 74 ist vorzugsweise mittels einer Verbindungsleitung mit der Dosiereinheit 76 verbunden. Die Dosiereinheit 76 ist insbesondere mittels einer Verbindungsleitung mit einem Zuführelement, insbesondere einer Zuführdüse oder einem Zuführtropfer, der Verdampfervorrichtung 54 verbunden. Bevorzugt ist zumindest ein Sensor 34 der Überwachungsvorrichtung 50 als Stoffkonzentrationssensor ausgebildet, der zu einem Erfassen einer Konzentration des Stoffs im flüssigen Zustand vorgesehen ist. Vorzugsweise ist zumindest ein Sensor 36 der Überwachungsvorrichtung 50 als Stofftemperatursensor ausgebildet, der zu einem Erfassen einer Temperatur des Stoffs im flüssigen Zustand vorgesehen ist. Insbesondere ist zumindest ein Sensor 38 der Überwachungsvorrichtung 50 als Stoffmengensensor ausgebildet, der zu einem Erfassen einer Menge/eines Volumens des Stoffs im flüssigen Zustand, insbesondere pro Takt des Sterilisationsprozesses, vorgesehen ist.

Die Überwachungsvorrichtung 50 umfasst zumindest einen Sensor 40, 42, insbesondere eine Vielzahl an Sensoren 40, 42, zu einer Erfassung einer die Verdampfervorrichtung 54 der Produktionsmaschine 12 definierenden Kenngröße. Insbesondere ist der Sensor 40, 42, insbesondere die Sensoren 40, 42, zu einer Erfassung der die Verdampfervorrichtung 54 definierenden Kenngröße an der Verdampfervorrichtung 54 angeordnet. Bevorzugt ist zumindest ein Sensor 40 der Überwachungsvorrichtung 50 als Verdampferheizeinheittemperatursensor ausgebildet, der zu einem Erfassen einer Temperatur einer Verdampferheizeinheit 56 (vgl. Figur 2) der Verdampfervorrichtung 54 vorgesehen ist. Vorzugsweise ist zumindest ein Sensor 42 der Überwachungsvorrichtung 50 als Verdampfereinschaltdauersensor ausgebildet, der zu einem Erfassen einer Einschaltdauer der Verdampfervorrichtung 54, insbesondere der Verdampferheizeinheit 56, vorgesehen ist.

Die Überwachungsvorrichtung 50 umfasst zumindest einen Sensor 44, 46, 48, insbesondere eine Vielzahl an Sensoren 44, 46, 48, zu einer Erfassung einer eine an einer Verdampferausgangsleitung 58 (vgl. Figur 2) der Produktionsmaschine 12 angeordnete Heizeinheit 60, 62 der Produktionsmaschine 12 definierenden Kenngröße. Insbesondere ist der Sensor 44, 46, 48, insbesondere die Sensoren 44, 46, 48, zu einer Erfassung der die an der Verdampferausgangsleitung 58 angeordneten Heizeinheit 60, 62 definierenden Kenngröße an der Heizeinheit 60, 62 angeordnet. Bevorzugt ist zumindest ein Sensor 44 der Überwachungsvorrichtung 50 als Heizeinheitentemperatursensor ausgebildet, der zu einem Erfassen einer Temperatur der Heizeinheit 60, 62, insbesondere zumindest einer als Wickelheizung ausgebildeten Heizeinheit 60 (vgl. Figur 2), vorgesehen ist. Vorzugsweise ist zumindest ein Sensor 46, 48 der Überwachungsvorrichtung 50 als Heizeinheiteneinschaltdauersensor ausgebildet, der zu einem Erfassen einer Einschaltdauer der Heizeinheit 60, 62, insbesondere einer Einschaltdauer der als Wickelheizung ausgebildeten Heizeinheit 60 und einer Einschaltdauer einer als Heizschlauch ausgebildeten weiteren Heizeinheit 62 (vgl. Figur 2), vorgesehen ist.

Figur 3 zeigt einen schematischen Ablauf eines Verfahrens 10 zu einer Bestimmung einer Stoffkonzentration, insbesondere einer H₂O₂-Konzentration, in einem Gasgemisch in dem Sterilisationsprozess der Produktionsmaschine 12. Vorzugsweise erfolgt in dem Verfahren 10 eine Ermittlung der oder ein Rückschluss auf die zu bestimmende/n Stoffkonzentration, insbesondere H₂O₂-Konzentration, im Gasgemisch anhand eines Abgleichs und/oder einer Auswertung des zumindest einen während eines aktuellen Betriebs erfassten umgebungs- und/oder prozessspezifischen Parameters, insbesondere der während eines aktuellen Betriebs erfassten umgebungs- und/oder prozessspezifischen Parameter, mit in dem mittels des maschinellen Lernprozesses erstellten Auswertemodell hinterlegten Daten. Vorzugsweise wird der Zusammenhang der zu bestimmenden Stoffkonzentration, insbesondere der zu bestimmenden H₂O₂-Konzentration, im Gasgemisch mit zumindest einem umgebungs- und/oder prozessspezifischen Parameter, insbesondere mit vielen umgebungs- und/oder prozessspezifischen Parametern, anhand von einer Vielzahl an im Vorfeld erfassten Daten in dem mittels des maschinellen Lernprozesses erstellten Auswertemodell erfasst, insbesondere in einem Datenaufzeichnungsbetrieb 78.

Das mittels des maschinellen Lernprozesses erstellte Auswertemodell wird vorzugsweise dadurch erstellt, dass während des Datenaufzeichnungsbetriebs 78 unterschiedliche Einstellungen der umgebungs- und/oder prozessspezifischen Parameter erfolgen und deren Auswirkung auf die Stoffkonzentration, insbesondere die H₂O₂-Konzentration, im Gasgemisch mittels des zumindest temporär installierten, physischen Stoffkonzentrationssensors 68 aufgezeichnet werden, insbesondere um im realen Betrieb, insbesondere Regelbetrieb, des Verfahrens 10 und/oder der Produktionsmaschine 12, insbesondere in einem frei von dem physischen Stoffkonzentrationssensor 68 ausgebildeten Betrieb des Verfahrens 10 und/oder der Produktionsmaschine 12, zuverlässige Rückschlüsse auf Werte für die Stoffkonzentration, insbesondere die H₂O₂-Konzentration, im Gasgemisch zu erhalten. Vorzugsweise erfolgt der Datenaufzeichnungsbetrieb 78 vor einem realen Betrieb, insbesondere vor einem Regelbetrieb, der Produktionsmaschine 12, wie beispielsweise während einer Installation der Produktionsmaschine 12 an einem für den realen Betrieb, insbesondere Regelbetrieb, vorgesehenen Standort der Produktionsmaschine 12 oder in einer Entwicklungsphase der Produktionsmaschine 12. Vorzugsweise ist der Datenaufzeichnungsbetrieb 78 für die Produktionsmaschine 12 nur einmal durchzuführen, insbesondere um das Auswertemodell mit Daten zu füllen. Bevorzugt werden zur Erstellung des Auswertemodells die, insbesondere während des Datenaufzeichnungsbetriebs 78, durchgeführten Einstellungen des/der umgebungs- und/oder prozessspezifische/n Parameter und Werte der Stoffkonzentration, die mittels des physischen Stoffkonzentrationssensors 68 aufgezeichnet werden, in Datensätzen für das Auswertemodell abgelegt. Bevorzugt greift das mittels des maschinellen Lernprozesses erstellte Auswertemodell in einem realen Betrieb, insbesondere Regelbetrieb, der Produktionsmaschine 12, insbesondere in einem frei von dem physischen Stoffkonzentrationssensor 68 zur Erfassung der Stoffkonzentration ablaufenden Betrieb, auf diese Datensätze zu und/oder gleicht die während eines realen Betriebs, insbesondere Regelbetriebs, der Produktionsmaschine 12 erfassten umgebungs- und/oder prozessspezifischen Parameter mit den in den Datensätzen hinterlegten Werten ab, insbesondere um auf die Stoffkonzentration, insbesondere die H₂O₂-Konzentration, im Gasgemisch in Abhängigkeit von erfassten umgebungs- und/oder prozessspezifischen Parametern zu schließen. Bevorzugt erfolgt nach dem Datenaufzeichnungsbetrieb 78 ein Implementierungsschritt 80, in dem das mittels des maschinellen Lernprozesses erstellte Auswertemodell in die Recheneinheit 70 der Überwachungsvorrichtung 50 implementiert wird. Vorzugsweise kann nach dem Implementierungsschritt 80 ein Regelbetrieb der Produktionsmaschine 12 durchgeführt werden, in dem das Verfahren 10 in einem realen Betrieb abläuft. In zumindest einem Verfahrensschritt 66 des Verfahrens 10, insbesondere vor einem Regelbetrieb der Produktionsmaschine 12 vorzugsweise nach dem Implementierungsschritt 80, erfolgt eine Kalibration mittels des, insbesondere physischen, Stoffkonzentrationssensors 68 (in Figur 2 nur gestrichelt dargestellt), insbesondere mittels eines H₂O₂-Konzentrationssensors. Der Stoffkonzentrationssensor 68 ist vorzugsweise ist der Verdampferausgangsleitung 58 angeordnet bzw. in diese integriert. Es ist denkbar, dass in einem alternativen oder zusätzlichen Verfahrensschritt 64 des Verfahrens 10 eine Justierung, insbesondere anhand der Kalibration, des Auswertemodells, insbesondere der hinterlegten Datensätze, erfolgt.

Das Verfahren 10 läuft in einem Regelbetrieb der Produktionsmaschine 12 vorzugsweise wie folgt ab:
In zumindest einem Verfahrensschritt 14 des Verfahrens 10 wird zumindest ein verschieden von der Stoffkonzentration ausgebildeter, umgebungs- und/oder prozessspezifischer Parameter mittels eines der Sensoren 16, 18, 20, 22, 24, 26, 28, 30, 32, 34, 36, 38, 40, 42, 44, 46, 48 der Überwachungsvorrichtung 50 der Produktionsmaschine 12 erfasst. In dem zumindest einen Verfahrensschritt 14 werden zumindest zwei, insbesondere zumindest vier, verschiedene umgebungs- und/oder prozessspezifische Parameter mittels der Sensoren 16, 18, 20, 22, 24, 26, 28, 30, 32, 34, 36, 38, 40, 42, 44, 46, 48 der Überwachungsvorrichtung 50 erfasst und dem mittels des maschinellen Lernprozesses erstellten Auswertemodell zur Auswertung zugeführt, um in Abhängigkeit von der Auswertung auf die Stoffkonzentration in dem Gasgemisch zu schließen. In zumindest einem Verfahrensschritt 52 des Verfahrens 10 wird/werden der zumindest eine/die erfasste/n umgebungs- und/oder prozessspezifische/n Parameter dem mittels des maschinellen Lernprozesses erstellten Auswertemodell zu einer Auswertung zugeführt und in Abhängigkeit von der Auswertung auf die Stoffkonzentration, insbesondere auf die H₂O₂-Konzentration, in dem Gasgemisch geschlossen.

Der umgebungs- und/oder prozessspezifische Parameter kann eine die Produktionsmaschine 12 umgebende Umgebungsluft definierende Kenngröße, insbesondere eine relative Luftfeuchte der Umgebungsluft, eine Temperatur der Umgebungsluft und/oder ein Luftdruck der Umgebungsluft, sein, die mittels eines der Sensoren 16, 18, 20, insbesondere eines Umgebungsluftfeuchtesensors, eines Umgebungstemperatursensors und/oder eines Umgebungsluftdrucksensors, der Überwachungsvorrichtung 50 erfasst wird. Der umgebungs- und/oder prozessspezifische Parameter kann die zu einer Förderung des flüssigen und/oder aerosolierten Stoffs, der zu einer Generierung des Gasgemischs verdampft wird, vorgesehene Druckluft definierende Kenngröße, insbesondere ein Volumenstrom der Druckluft, eine Temperatur der Druckluft, ein Druck der Druckluft, ein Sättigungsdampfdruck der Druckluft, ein Dampfanteil der Druckluft und/oder eine absolute Luftfeuchte der Druckluft, sein, die mittels eines der Sensoren 22, 24, 26, 28, 30, 32, insbesondere eines Druckluftvolumenstromsensors, eines Drucklufttemperatursensors, eines Druckluftdrucksensors, eines Druckluftsättigungsdampfdrucksensors, eines Druckluftdampfanteilsensors und/oder eines Druckluftfeuchtesensors, der Überwachungsvorrichtung 50 erfasst wird. Der umgebungs- und/oder prozessspezifische Parameter kann eine den flüssigen Stoff, der zur Generierung des Gasgemischs verdampft wird, definierende Kenngröße, insbesondere eine Konzentration des Stoffs im flüssigen Zustand, eine Temperatur des Stoffs im flüssigen Zustand und/oder eine Menge/Volumen des Stoffs im flüssigen Zustand pro Takt des Sterilisationsprozesses, sein, die mittels eines der Sensoren 34, 36, 38, insbesondere eines Stoffkonzentrationssensors, eines Stofftemperatursensors und/oder eines Stoffmengensensors, der Überwachungsvorrichtung 50 erfasst wird. Der umgebungs- und/oder prozessspezifische Parameter kann eine die Verdampfervorrichtung 54 definierende Kenngröße, insbesondere eine Temperatur der Verdampferheizeinheit 56 und/oder eine Einschaltdauer der Verdampfervorrichtung 54, sein, die mittels eines der Sensoren 40, 42, insbesondere eines Verdampferheizeinheittemperatursensors und/oder eines Verdampfereinschaltdauersensors, der Überwachungsvorrichtung 50 erfasst wird. Der umgebungs- und/oder prozessspezifische Parameter kann eine die an der Verdampferausgangsleitung 58 angeordnete Heizeinheit 60, 62 definierende Kenngröße, insbesondere eine Temperatur der Heizeinheit 60, 62 und/oder eine Einschaltdauer der Heizeinheit 60, 62, sein, die mittels eines der Sensoren 44, 46, 48, insbesondere eines Heizeinheitentemperatursensors und/oder eines Heizeinheiteneinschaltdauersensors, der Überwachungsvorrichtung 50 erfasst wird.

## Patentansprüche

1. Verfahren zu einer Bestimmung einer Stoffkonzentration, insbesondere einer H₂O₂-Konzentration, in einem Gasgemisch in einem Sterilisationsprozess einer Produktionsmaschine, wobei in zumindest einem Verfahrensschritt (14) zumindest ein verschieden von der Stoffkonzentration ausgebildeter umgebungs- und/oder prozessspezifischer Parameter mittels eines Sensors (16, 18, 20, 22, 24, 26, 28, 30, 32, 34, 36, 38, 40, 42, 44, 46, 48) einer Überwachungsvorrichtung der Produktionsmaschine erfasst wird, und wobei in zumindest einem Verfahrensschritt (52) der zumindest eine erfasste umgebungs- und/oder prozessspezifische Parameter einem mittels eines maschinellen Lernprozesses erstellten Auswertemodell zu einer Auswertung zugeführt wird und in Abhängigkeit von der Auswertung auf die Stoffkonzentration, insbesondere auf die H₂O₂-Konzentration, in dem Gasgemisch geschlossen wird.

2. Verfahren nach Anspruch 1, **dadurch gekennzeichnet, dass** in dem zumindest einen Verfahrensschritt (14) zumindest zwei, insbesondere zumindest vier, verschiedene umgebungs- und/oder prozessspezifische Parameter mittels Sensoren (16, 18, 20, 22, 24, 26, 28, 30, 32, 34, 36, 38, 40, 42, 44, 46, 48) der Überwachungsvorrichtung erfasst werden und dem mittels des maschinellen Lernprozesses erstellten Auswertemodell zur Auswertung zugeführt werden, um in Abhängigkeit von der Auswertung auf die Stoffkonzentration in dem Gasgemisch zu schließen.

3. Verfahren nach Anspruch 1 oder 2, **dadurch gekennzeichnet, dass** der umgebungs- und/oder prozessspezifische Parameter eine die Produktionsmaschine umgebende Umgebungsluft definierende Kenngröße, insbesondere eine relative Luftfeuchte der Umgebungsluft, eine Temperatur der Umgebungsluft und/oder ein Luftdruck der Umgebungsluft, ist, die mittels eines Sensors (16, 18, 20), insbesondere eines Umgebungsluftfeuchtesensors, eines Umgebungstemperatursensors und/oder eines Umgebungsluftdrucksensors, der Überwachungsvorrichtung erfasst wird.

4. Verfahren nach einem der vorhergehenden Ansprüche, **dadurch gekennzeichnet, dass** der umgebungs- und/oder prozessspezifische Parameter eine zu einer Förderung eines flüssigen und/oder aerosolierten Stoffs, der zu einer Generierung des Gasgemischs verdampft wird, vorgesehene Druckluft definierende Kenngröße, insbesondere ein Volumenstrom der Druckluft, eine Temperatur der Druckluft, ein Druck der Druckluft, ein Sättigungsdampfdruck der Druckluft, ein Dampfanteil der Druckluft und/oder eine absolute Luftfeuchte der Druckluft, ist, die mittels eines Sensors (22, 24, 26, 28, 30, 32), insbesondere eines Druckluftvolumenstromsensors, eines Drucklufttemperatursensors, eines Druckluftdrucksensors, eines Druckluftsättigungsdampfdrucksensors, eines Druckluftdampfanteilsensors und/oder eines Druckluftfeuchtesensors, der Überwachungsvorrichtung erfasst wird.

5. Verfahren nach einem der vorhergehenden Ansprüche, **dadurch gekennzeichnet, dass** der umgebungs- und/oder prozessspezifische Parameter eine einen flüssigen Stoff, der zur Generierung des Gasgemischs verdampft wird, definierende Kenngröße, insbesondere eine Konzentration des Stoffs im flüssigen Zustand, eine Temperatur des Stoffs im flüssigen Zustand und/oder eine Menge/Volumen des Stoffs im flüssigen Zustand pro Takt des Sterilisationsprozesses, ist, die mittels eines Sensors (34, 36, 38), insbesondere eines Stoffkonzentrationssensors, eines Stofftemperatursensors und/oder eines Stoffmengensensors, der Überwachungsvorrichtung erfasst wird.

6. Verfahren nach einem der vorhergehenden Ansprüche, **dadurch gekennzeichnet, dass** der umgebungs- und/oder prozessspezifische Parameter eine eine Verdampfervorrichtung (54) der Produktionsmaschine definierende Kenngröße, insbesondere eine Temperatur einer Verdampferheizeinheit (56) der Verdampfervorrichtung (54) und/oder eine Einschaltdauer der Verdampfervorrichtung (54), ist, die mittels eines Sensors (40, 42), insbesondere eines Verdampferheizeinheittemperatursensors und/oder eines Verdampfereinschaltdauersensors, der Überwachungsvorrichtung erfasst wird.

7. Verfahren nach einem der vorhergehenden Ansprüche, **dadurch gekennzeichnet, dass** der umgebungs- und/oder prozessspezifische Parameter eine eine an einer Verdampferausgangsleitung (58) der Produktionsmaschine angeordnete Heizeinheit (60, 62) der Produktionsmaschine definierende Kenngröße, insbesondere eine Temperatur der Heizeinheit (60, 62) und/oder eine Einschaltdauer der Heizeinheit (60, 62), ist, die mittels eines Sensors (44, 46, 48), insbesondere eines Heizeinheitentemperatursensors und/oder eines Heizeinheiteneinschaltdauersensors, der Überwachungsvorrichtung erfasst wird.

8. Verfahren nach einem der vorhergehenden Ansprüche, **dadurch gekennzeichnet, dass** in zumindest einem Verfahrensschritt (66), insbesondere vor einem Regelbetrieb der Produktionsmaschine, eine Kalibration mittels eines, insbesondere physischen, Stoffkonzentrationssensors (68), insbesondere mittels eines H₂O₂-Konzentrationssensors, erfolgt.

9. Überwachungsvorrichtung zu einer Überwachung einer Stoffkonzentration, insbesondere einer H₂O₂-Konzentration, in einem Gasgemisch in einem Sterilisationsprozess einer Produktionsmaschine, mit zumindest einer Recheneinheit (70) zu einem Ausführen des Verfahrens nach einem der vorhergehenden Ansprüche und mit zumindest einem Sensor (16, 18, 20, 22, 24, 26, 28, 30, 32, 34, 36, 38, 40, 42, 44, 46, 48) zu einem Erfassen zumindest eines verschieden von der Stoffkonzentration ausgebildeten umgebungs- und/oder prozessspezifischen Parameters, der zu einem Übertragen von Daten mit der Recheneinheit (70) verbunden ist, **dadurch gekennzeichnet, dass** die Recheneinheit (70) zumindest einen Speicher (72) aufweist, in dem ein mittels eines maschinellen Lernprozesses erstelltes Auswertemodell abgelegt ist, das dazu eingerichtet ist, in Abhängigkeit von einer Auswertung des zumindest einen erfassten umgebungs- und/oder prozessspezifischen Parameters auf die Stoffkonzentration, insbesondere auf die H₂O₂-Konzentration, in dem Gasgemisch zu schließen, insbesondere frei von einem physischen Stoffkonzentrationssensor (68).

10. Produktionsmaschine, insbesondere Lebensmittelabfüll- und/oder Lebensmittelverpackungsmaschine, mit zumindest einer Überwachungsvorrichtung nach Anspruch 9.

## Claims

1. Method for determining a substance concentration, in particular an H₂O₂ concentration, in a gas mixture in a sterilization process of a production machine, wherein in at least one method step (14) at least one environment-specific and/or process-specific parameter which is different from the substance concentration is detected by means of a sensor (16, 18, 20, 22, 24, 26, 28, 30, 32, 34, 36, 38, 40, 42, 44, 46, 48) of a monitoring device of the production machine, and wherein in at least one method step (52) the at least one detected environment-specific and/or process-specific parameter is supplied to an evaluation model, which is created by means of a machine learning process, for evaluation and the substance concentration, in particular the H₂O₂ concentration, in the gas mixture is inferred as a function of the evaluation.

2. Method according to claim 1, **characterized in that** in the at least one method step (14) at least two, in particular at least four, different environment-specific and/or process-specific parameters are detected by means of sensors (16, 18, 20, 22, 24, 26, 28, 30, 32, 34, 36, 38, 40, 42, 44, 46, 48) of the monitoring device and are supplied to the evaluation model, which is created by means of the machine learning process, for evaluation in order to infer the substance concentration in the gas mixture as a function of the evaluation.

3. Method according to claim 1 or 2, **characterized in that** the environment-specific and/or process-specific parameter is a characteristic variable which defines the ambient air surrounding the production machine, in particular a relative humidity of the ambient air, a temperature of the ambient air and/or an air pressure of the ambient air, which is detected by means of a sensor (16, 18, 20), in particular an ambient humidity sensor, an ambient temperature sensor and/or an ambient air pressure sensor, of the monitoring device.

4. Method according to one of the preceding claims, **characterized in that** the environment-specific and/or process-specific parameter is a characteristic variable which defines compressed air which is provided for conveying a liquid and/or aerosolized substance which is vaporized in order to generate the gas mixture, in particular a volume flow of the compressed air, a temperature of the compressed air, a pressure of the compressed air, a saturation vapour pressure of the compressed air, a vapour fraction of the compressed air and/or an absolute humidity of the compressed air, which is detected by means of a sensor (22, 24, 26, 28, 30, 32), in particular a compressed air volume flow sensor, a compressed air temperature sensor, a compressed air pressure sensor, a compressed air saturation vapour pressure sensor, a compressed air vapour fraction sensor and/or a compressed air humidity sensor, of the monitoring device.

5. Method according to one of the preceding claims, **characterized in that** the environment-specific and/or process-specific parameter is a characteristic variable which defines a liquid substance which is vaporized in order to generate the gas mixture, in particular a concentration of the substance in the liquid state, a temperature of the substance in the liquid state and/or a quantity/volume of the substance in the liquid state per cycle of the sterilization process, which is detected by means of a sensor (34, 36, 38), in particular a substance concentration sensor, a substance temperature sensor and/or a substance quantity sensor, of the monitoring device.

6. Method according to one of the preceding claims, **characterized in that** the environment-specific and/or process-specific parameter is a characteristic variable which defines a vaporizer device (54) of the production machine, in particular a temperature of a vaporizer heating unit (56) of the vaporizer device (54) and/or a switch-on duration of the vaporizer device (54), which is detected by means of a sensor (40, 42), in particular a vaporizer heating unit temperature sensor and/or a vaporizer switch-on duration sensor, of the monitoring device.

7. Method according to one of the preceding claims, **characterized in that** the environment-specific and/or process-specific parameter is a characteristic variable which defines a heating unit (60, 62) of the production machine, which heating unit (60, 62) is arranged on a vaporizer outlet line (58) of the production machine, in particular a temperature of the heating unit (60, 62) and/or a switch-on duration of the heating unit (60, 62), which is detected by means of a sensor (44, 46, 48), in particular a heating unit temperature sensor and/or a heating unit switch-on duration sensor, of the monitoring device.

8. Method according to one of the preceding claims, **characterized in that,** in at least one method step (66), in particular before a regular operation of the production machine, a calibration is carried out by means of a, in particular physical, substance concentration sensor (68), in particular by means of an H₂O₂ concentration sensor.

9. Monitoring device for monitoring a substance concentration, in particular an H₂O₂ concentration, in a gas mixture in a sterilization process of a production machine, having at least one computing unit (70) for carrying out the method according to one of the preceding claims and having at least one sensor (16, 18, 20, 22, 24, 26, 28, 30, 32, 34, 36, 38, 40, 42, 44, 46, 48) for detecting at least one environment-specific and/or process-specific parameter which is different from the substance concentration and which is connected to the computing unit (70) for transmitting data, **characterized in that** the computing unit (70) has at least one memory (72) in which an evaluation model which is created by means of a machine learning process and which is set up to infer the substance concentration, in particular the H₂O₂ concentration, in the gas mixture, in particular free of a physical substance concentration sensor (68), as a function of an evaluation of the at least one detected environment-specific and/or process-specific parameter, is stored.

10. Production machine, in particular food filling machine and/or food packaging machine, having at least one monitoring device according to claim 9.

## Revendications

1. Procédé de détermination d'une concentration de substance, en particulier d'une concentration en H₂O₂, dans un mélange gazeux dans un processus de stérilisation d'une machine de production, où, dans au moins une étape de procédé (14), au moins un paramètre spécifique à l'environnement et/ou au processus, réalisé différemment de la concentration de substance, est détecté au moyen d'un capteur (16, 18, 20, 22, 24, 26, 28, 30, 32, 34, 36, 38, 40, 42, 44, 46, 48) d'un dispositif de surveillance de la machine de production, et où, dans au moins une étape de procédé (52), l'au moins un paramètre spécifique à l'environnement et/ou au processus détecté est amené à un modèle d'évaluation établi au moyen d'un processus d'apprentissage machine pour une évaluation et, en fonction de l'évaluation, on conclut à la concentration de substance, en particulier à la concentration en H₂O₂, dans le mélange gazeux.

2. Procédé selon la revendication 1, **caractérisé en ce que,** dans l'au moins une étape de procédé (14), au moins deux, en particulier au moins quatre, paramètres différents spécifiques à l'environnement et/ou au processus sont détectés au moyen de capteurs (16, 18, 20, 22, 24, 26, 28, 30, 32, 34, 36, 38, 40, 42, 44, 46, 48) du dispositif de surveillance et sont amenés au modèle d'évaluation établi au moyen du processus d'apprentissage machine pour l'évaluation, afin de conclure à la concentration de substance dans le mélange gazeux en fonction de l'évaluation.

3. Procédé selon la revendication 1 ou 2, **caractérisé en ce que** le paramètre spécifique à l'environnement et/ou au processus est une grandeur caractéristique définissant l'air ambiant entourant la machine de production, en particulier une humidité relative de l'air ambiant, une température de l'air ambiant et/ou une pression d'air de l'air ambiant, qui est détectée au moyen d'un capteur (16, 18, 20), en particulier d'un capteur d'humidité de l'air ambiant, d'un capteur de température ambiante et/ou d'un capteur de pression d'air ambiante, du dispositif de surveillance.

4. Procédé selon l'une quelconque des revendications précédentes, **caractérisé en ce que** le paramètre spécifique à l'environnement et/ou au processus est une grandeur caractéristique prévue pour un transport d'une substance liquide et/ou en aérosol, qui est vaporisée pour une génération du mélange gazeux, en particulier un débit volumique de l'air comprimé, une température de l'air comprimé, une pression de l'air comprimé, une pression de vapeur saturante de l'air comprimé, une proportion de vapeur de l'air comprimé et/ou une humidité absolue de l'air comprimé, qui est détectée au moyen d'un capteur (22, 24, 26, 28, 30, 32), en particulier d'un capteur de débit volumique d'air comprimé, d'un capteur de température d'air comprimé, d'un capteur de pression d'air comprimé, d'un capteur de pression de vapeur saturante d'air comprimé, d'un capteur de proportion de vapeur d'air comprimé et/ou d'un capteur d'humidité d'air comprimé, du dispositif de surveillance.

5. Procédé selon l'une quelconque des revendications précédentes, **caractérisé en ce que** le paramètre spécifique à l'environnement et/ou au processus est une grandeur caractéristique définissant une substance liquide, qui est vaporisée pour la génération du mélange gazeux, en particulier une concentration de la substance à l'état liquide, une température de la substance à l'état liquide et/ou une quantité/volume de la substance à l'état liquide par cycle du processus de stérilisation, qui est détectée au moyen d'un capteur (34, 36, 38), en particulier d'un capteur de concentration de substance, d'un capteur de température de substance et/ou d'un capteur de quantité de substance, du dispositif de surveillance.

6. Procédé selon l'une quelconque des revendications précédentes, **caractérisé en ce que** le paramètre spécifique à l'environnement et/ou au processus est une grandeur caractéristique définissant un dispositif évaporateur (54) de la machine de production, en particulier une température d'une unité de chauffage d'évaporateur (56) du dispositif évaporateur (54) et/ou une durée de service du dispositif évaporateur (54), qui est détectée au moyen d'un capteur (40, 42), en particulier d'un capteur de température d'unité de chauffage d'évaporateur et/ou d'un capteur de durée de service d'évaporateur, du dispositif de surveillance.

7. Procédé selon l'une quelconque des revendications précédentes, **caractérisé en ce que** le paramètre spécifique à l'environnement et/ou au processus est une grandeur caractéristique définissant une unité de chauffage (60, 62) de la machine de production disposée au niveau d'une conduite de sortie d'évaporateur (58) de la machine de production, en particulier une température de l'unité de chauffage (60, 62) et/ou une durée de service de l'unité de chauffage (60, 62), qui est détectée au moyen d'un capteur (44, 46, 48), en particulier d'un capteur de température d'unité de chauffage et/ou d'un capteur de durée de service d'unité de chauffage, du dispositif de surveillance.

8. Procédé selon l'une quelconque des revendications précédentes, **caractérisé en ce que** dans au moins une étape de procédé (66), en particulier avant un fonctionnement régulé de la machine de production, un calibrage a lieu au moyen d'un capteur de concentration de substance (68), en particulier physique, en particulier au moyen d'un capteur de concentration de H₂O₂.

9. Dispositif de surveillance pour une surveillance d'une concentration de substance, en particulier d'une concentration de H₂O₂ , dans un mélange gazeux dans un processus de stérilisation d'une machine de production, comprenant au moins une unité de calcul (70) pour une exécution du procédé selon l'une quelconque des revendications précédentes et comprenant au moins un capteur (16, 18, 20, 22, 24, 26, 28, 30, 32, 34, 36, 38, 40, 42, 44, 46, 48) pour une détection d'au moins un paramètre spécifique à l'environnement et/ou au processus, réalisé différemment de la concentration de substance, qui est relié à l'unité de calcul (70) pour une transmission de données, **caractérisé en ce que** l'unité de calcul (70) présente au moins une mémoire (72), dans laquelle est stocké un modèle d'évaluation établi au moyen d'un processus d'apprentissage machine, qui est conçu pour, en fonction d'une évaluation de l'au moins un paramètre spécifique à l'environnement et/ou au processus détecté, conclure à la concentration de substance, en particulier à la concentration de H₂O₂ , dans le mélange gazeux, en particulier exempt de capteur de concentration de substance physique (68).

10. Machine de production, en particulier machine de remplissage d'aliments et/ou d'emballage d'aliments, comprenant au moins un dispositif de surveillance selon la revendication 9.
